# EUROPÄISCHE PATENTSCHRIFT

(11) **EP 0 974 050 B1**
(45) Veröffentlichungstag und Bekanntmachung des Hinweises auf die Patenterteilung: **25.09.2002**
(21) Anmeldenummer: 97901071.7
(22) Anmeldetag: 20.01.1997
(51) Int. Cl.: G01N 33/50, G01N 33/68, G01N 33/566, G01N 33/58, G01N 1/30

(54) **BASOPHILEN-DEGRANULATIONSTEST**
BASOPHIL DEGRANULATION TEST
TEST DE DEGRANULATION DES BASOPHILES

(43) Veröffentlichungstag der Anmeldung: 26.01.2000
(73) Patentinhaber: ORPEGEN Pharma GmbH, 69115 Heidelberg (DE)
(72) Erfinder: NEBE, Carl, Thomas, D-68526 Ladenburg (DE); HARTMANN, Karin, D-67158 Ellerstadt (DE)
(74) Vertreter: Weiss, Wolfgang, Dipl.-Chem. Dr.
(86) Internationale Anmeldenummer: EP9700251
(87) Internationale Veröffentlichungsnummer: WO98032014

(56) Entgegenhaltungen:
- EP-A- 0 022 698
- EP-A- 0 185 335
- WO-A-84/04109
- WO-A-93/25904
- WO-A-95/15494
- FR-A- 2 467 401

## Beschreibung

Die vorliegende Erfindung betrifft ein Verfahren zur Bestimmung der Degranulationsaktivität von basophilen Granulozyten in einer Probe sowie ein dazu geeignetes Reagenzienkit. Weiterhin betrifft die Erfindung auch ein Diagnoseverfahren auf allergische Hypersensibilität und den Erfolg einer Hyposensibilisierung.

Die klassische Einteilung der Allergien nach Coombs und Gell gilt heute noch weitgehend als Grundlage zum Verständnis der allergischen Typ-1-Reaktion vom Soforttyp und ist klar definiert durch eine IgE-vermittelte Immunantwort. Dabei spielen T-Zellen mit einem bestimmten Zytokinmuster (Interleukin-4 und -5) eine besondere Rolle. B-Lymphozyten werden dadurch zu einer Umschaltung auf IgE-Produktion bewegt, welches nur in sehr niedrigen Spiegeln im Serum vorkommt und erst nach Zellbindung über hochaffine Rezeptoren durch deren Degranulation der Zellen seine fatale Wirkung entfaltet. Die Zytokine, insbesondere Interleukin-5, bewirken außerdem eine vermehrte Ausreifung von Promyelozyten in eosinophile Granulozyten, die durch die Freisetzung toxischer Proteine aus ihren Granula Spätschäden insbesondere am Bronchialsystem verursachen. Die physiologische Bedeutung der Typ-1-Allergie ist noch völlig unklar, ebenso wie die Ursache, warum die T-Zellen ein Zytokinmuster für eine IgE-Antwort sezernieren.

Die Bestimmung von spezifischen IgE-Antikörpern im Serum (RAST, CAP u.ä.) ist gut etabliert und insbesondere dann hilfreich, wenn bekannte Allergene zu prüfen sind oder wenn eine Hauttestung gefährlich oder technisch nicht möglich ist, wie z. B. bei Hauterkrankungen oder unter Medikamenteneinfluß. Allergene werden dabei kovalent oder mit hoher Affinität an Matrices mit großer Oberfläche (Schwämmchen, Kügelchen, Papierscheiben) gebunden, anschließend mit Patientenserum inkubiert und dann nach Waschen wird eine Detektion mit einem radioaktiven oder enzymmarkierten anti-human-IgE-Antikörper durchgeführt. Die Menge des gebundenen IgE wird semiquantitativ in sog. RAST- bzw. CAP-Klassen oder quantitativ in kU IgE/1 angegeben. Die Auswertung kann radiometrisch (RAST) oder z. B. fluorimetrisch (CAP, Enzym mit fluorogenem substrat) erfolgen.

Die Vorteile dieses Tests sind die leichte Durchführbarkeit und eine gute Korrelation mit dem Goldstandard Hauttest bei Aeroallergenen bzw. den sog. Atopenen. Allerdings müssen dafür schwerwiegende Nachteile in Kauf genommen werden. Diese Nachteile sind z. B. die mangelnde Verfügbarkeit von seltenen Allergenen und oft die Epitopveränderungen durch Kopplung der Allergene an die Matrix. Außerdem wird bei kleineren Allergenen (Haptenen) ein Carriermolekül wie z. B. humanes Serumalbumin benötigt. Weitere Schwierigkeiten werden dadurch verursacht, daß die Spezifitäten von Serum-IgE und membrangebundenem IgE auf Basophilen und Mastzellen nicht übereinstimmen müssen, da nur ein langsamer Austausch stattfindet. Auch wird die Schwelle der Degranulationsbereitschaft (releasability) nicht erfaßt. Die Korrelation mit dem Erfolg einer Hyposensibilisierung in Form eines IgE-Rückgangs ist nur bei Insektengiftallergenen einigermaßen befriedigend, d.h. in der Mehrzahl der Fälle fehlt die Erklärung. Weiterhin muß bei Atopikern mit hohem IgE-Spiegel eine Relativierung der spezifischen IgE-Werte erfolgen, d.h. der Gesamt-IgE Spiegel des Patienten muß bekannt sein. Die Schwere des Krankheitsbildes korreliert kaum mit der Höhe des spezifischen IgE-Spiegels.

Als vereinfachte Version dieses Verfahrens sind fertige Teststreifen entwickelt worden, auf denen verschiedenen Allergene aufgebracht sind. Die Auswertung erfolgt durch Vergleich der entstehenden Blaufärbung mit einer Farbskala. Inhalationsantigene, die eine Reaktion in den höheren RAST-Klassen erzeugen, werden gut erkannt. Ansonsten liegen wenig Vergleichsdaten vor. Der Test ist in erster Linie für niedergelassene Allergologen ohne größeres eigenes Labor gedacht.

Aufgrund der vorstehend genannten Probleme bei der Bestimmung von spezifischem Serum-IgE wurden Tests entwickelt, bei denen nach einer Stimulation mit einem Allergen eine Degranulation der basophilen Granulozyten bewirkt wird und danach die freigesetzten Inhaltsstoffe der Granula im Überstand bestimmt werden. Derartige Inhaltsstoffe sind z. B. Histamin (Fa. Immunotech, Fa. IBL), Leukotriene (CAST-ELISA, Fa. Biermann) oder Tryptase (Fa. Pharmacia). Diese Assays sind ausgelegt als Enzymimmunoassay im Batchverfahren und erfordern aufgrund von Inkubationsschritten über Nacht eine Dauer von zwei Tagen. Ein weiterer Nachteil in Bezug auf die Histaminbestimmung ist der Acylierungsschritt für das freigesetzte Histamin sowie ein oftmals überhöhter Histamingehalt der Allergenlösungen durch bakterielle Verunreinigungen. Weiterhin ist es aus wirtschaftlichen Gesichtspunkten erforderlich, die Überstände mehrerer Patienten zu sammeln, wodurch es zu einer Befundverzögerung kommt. Die Degranulation muß jedoch noch am Abnahmetag aus frischem Vollblut erfolgen.

Es hat daher bereits Versuche gegeben, die Degranulation zytometrisch nachzuweisen. In einem Ansatz wurde die Absorption von basophilen Granula mit einem Technicon H6000 Meßgerät bestimmt. Dabei werden am Bildschirm die Meßpunkte von Hand ausgezählt (Nilsson, Eur. J. Haematol, 45 suppl 53, 50-54, 1990). Alternativ erfolgt eine Messung des axialen Lichtverlusts nach Färbung mit Toluidinblau in einem Ortho Cytofluorographen (Nakagawa et al. (Allergy 36, 39-47, 1981). Ein weiterer Ansatz besteht darin, ein durchflußzytometrisches Zweifarben-Immunfluoreszenzverfahren durchzuführen, wobei unterschiedlich markierte anti-CD45- und anti-IgE-Antikörper verwendet werden (Gane et al., Cytometry 19, 361-365, 1995). Dieses Verfahren beruht auf einer relativen Erhöhung der Expressionsdichte von CD45 und einer Verringerung der IgE-Antikörperexpression bei Degranulation. Dieses Verfahren hat jedoch den Nachteil, daß die Änderung der CD45 Expressionsdichte nur gering ist.

Keines dieser zytometrischen Verfahren hat aufgrund der genannten Nachteile bisher Eingang in die Routine gefunden, da sie an bestimmte, nicht allgemein verfügbare Geräte gebunden sind oder die Signalunterschiede sehr gering sind. Ferner ist die Messung der Basophilen schwierig, da sie im Blut selten sind und allein aufgrund der Streulichteigenschaften nur schwer von den anderen Leukozyten unterschieden werden können.

Die der vorliegenden Erfindung zugrundeliegenden Aufgabe be-stand somit darin, ein neues Verfahren zur Bestimmung der Degranulationsaktivität von basophilen Granulozyten bereitzustellen, das einfach und rasch durchzuführen ist und mit herkömmlichen Durchflußzytometern, die in Krankenhäusern bzw. den Laborpraxen weitverbreitet sind, ausgeführt werden kann.

Diese Aufgabe wird gelöst durch ein Verfahren zur Bestimmung der Degranulationsaktivität von basophilen Granulozyten in einer Probe, wobei man
a) die Probe mit einer Testsubstanz vermischt und inkubiert,
b) die Probe mit einem ersten Bindemolekül, das mit zellständigen IgE-Antikörpern oder deren hochaffinen Rezeptoren bindefähig ist, und einem zweiten Bindemolekül, das mit einem Oberflächenmolekül, welches bei Degranulation de novo an der Oberfläche von basophilen Granulozyten erscheint, bindefähig ist, vermischt und inkubiert, wobei das erste Bindemolekül und das zweite Bindemolekül unterschiedliche nebeneinander nachweisbare Markierungsgruppen tragen oder jeweils mit unterschiedlichen nebeneinander nachweisbaren Markierungsgruppen bindefähig sind, und
c) die Markierungen der Bindemoleküle separat bestimmt und auf Basis dieser Bestimmung die Degranulationsaktivität in der Probe qualitativ oder/und quantitativ ermittelt. Bei dem erfindungsgemäßen Verfahren werden zunächst die Basophilen über die Markierung mit einem ersten spezifischen Bindemolekül, z. B. einem Antikörper eindeutig bestimmt. Dieses erste Bindemolekül bindet an zellständige IgE-Antikörper oder deren hochaffinen Rezeptoren. Nur Basophile besitzen eine hohe Oberflächendichte an IgE-Molekülen, die gebunden an hochaffine Rezeptoren (Pc,R1) vorliegen. Vorzugsweise werden solche IgEoder Rezeptor-spezifischen Antikörper verwendet, die nicht zu einer Vernetzung des IgE führen.

Die Besonderheit des erfindungsgemäßen Verfahrens besteht weiterhin darin, daß ein zweites Bindemolekül verwendet wird, das mit einem erst durch die Degranulation an der Oberfläche erscheinenden Antigen (de novo-Expression) bindefähig ist. Ein Beispiel für ein solches Antigen ist das präformierte lysosomale Protein gp55, das erst durch die Fusion der Granulamembran mit der Cytoplasmamembran im Rahmen der Degranulation an der Oberfläche erscheint und dort nachgewiesen werden kann.

Mit dem Begriff "Testsubstanz" wird in der vorliegenden Erfindung jedes Molekül verstanden, das eine Aktivierung von basophilen Granulozyten bzw. die Degranulation hervorrufen kann. Im allgemeinen ist die Testsubstanz ein Allergen wie etwa Pflanzenpollen, ein Insektengift, wie etwa Bienen- oder Wespengift, ein Medikament, wie etwa Penicillin oder ein Latexallergen. Außerdem werden mit "Testsubstanz" auch andere biologische und synthetische Substanzen bezeichnet, die eine derartige Aktivierung hervorrufen können und im Sinne einer Positivkontrolle verwendet werden können. Ein Beispiel dafür ist das chemotaktische Peptid fMLP.

In einer Ausführungsform des erfindungsgemäßen Verfahrens wird die Probe mit einem die Degranulationsaktivität verstärkenden Stimulans, z. B. einem Zytokin vorinkubiert. Die Menge des Zytokins beträgt vorzugsweise 0,1-100 ng pro Ansatz. Diese Vorinkubation führt zur einer Sensibilisierung der basophilen Granulozyten, wodurch die Schwelle der Degranulationsbereitschaft (releasability) herabgesetzt wird. Das verwendete Stimulans ist vorzugsweise ein Lymphokin wie etwa ein Interleukin oder ein Wachstumsfaktor wie etwa ein koloniestimulierender Faktor (CSF). Bevorzugt sind IL-3 oder GM-CSF. Die Vorinkubation mit dem Zytokin erfolgt üblicherweise vor Schritt (a) des erfindungsgemäßen Verfahrens d. h. vor der Zugabe des Allergens, kann jedoch auch gleichzeitig der Allergen-Inkubation durchgeführt werden.

Die Inkubation mit einem Allergen als Testsubstanz führt bei einer aus einem Allergiepatienten stammenden Probe zu einer Aktivierung der basophilen Granulozyten und somit zu einer Degranulation, deren Ausmaß mit dem Ausmaß der allergischen Reakion korreliert. Das erste Bindemolekül dient zur Definition der basophilen Granulozyten, und das zweite Bindemolekül dient zur Definition desjenigen Anteils an basophilen Granulozyten, bei dem eine Degranulation stattgefunden hat. Der relative Anteil der degranulierten Granulozyten kann somit auf einfache Weise bestimmt werden durch das Verhältnis der Zellen, die für das erste und zweite Bindemolekül positiv sind, zu den Zellen, die lediglich für das erste Bindemolekül positiv sind.

Das vom zweiten Bindemolekül erkannte Antigen ist üblicherweise ein Protein oder ein Glykoprotein, das erst aufgrund der Degranulation an der Oberfläche erscheint und somit eine eindeutige Unterscheidung zwischen degranulierten und nichtdegranulierten Granulozyten erlaubt. Bevorzugt ist das Oberflächenmolekül gp55.

Als erstes und zweites Bindemolekül können beliebige biologische Substanzen verwendet werden, sofern sie eine spezifische Bindung entweder mit IgE-Antikörpern oder deren hochaffinen Rezeptoren bzw. einem degranulationsspezifischen Oberflächenmolekül zeigen. Vorzugsweise werden als erstes oder/und zweites Bindemolekül ein Antikörper oder ein Antikörperfragment verwendet. Dabei sind monoklonale Antikörper bevorzugt, jedoch auch polyklonale Antikörper sind geeignet. Insbesondere für das erste Bindemolekül sind solche Antikörper oder Antikörperfragmente geeignet, die eine hohe Spezifität, aber geringe Vernetzungs- bzw. Degranulationseigenschaften aufweisen (z. B. Fab-Fragmente). Antikörper oder Antikörperfragmente gegen den FC_{∈}-Rezeptor sind besonders als erste Bindemoleküle geeignet, da bei einem geringen Teil der Patienten die Zellen durch anti-IgE nur unzureichend erkannt bzw. aktiviert werden.

Weiterhin ist es im erfindungsgemäßen Verfahren bevorzugt, nach einer definierten Inkubationsdauer mit dem Allergen und dem ersten und zweiten Bindemolekül die Degranulation abzustoppen. Dies kann z. B. durch eine Fixierung der Leukozyten erfolgen. Weiterhin ist es bevorzugt, nach der Inkubation die Erythrozyten zu lysieren. Eine Lyse der Erythrozyten und Fixierung der Leukozyten kann gegebenenfalls auch in einem Schritt erfolgen. So wird der Probe eine Lösung zugegeben, die beispielsweise eine hohe Konzentration von Ammoniumchlorid sowie ETDA enthält. Geeignet sind auch Lösungen, die Formaldehyd und/oder die Ethylengylykol enthalten. Eine bevorzugte Lösung zur Durchführung der Lyse und Fixierung ist eine unter der Handelsbezeichnung FACS-Lysing Solution (Becton Dickinson) kommerziell erhältliche Lösung. Gegebenenfalls wird im Anschluß an die Fixierung ein Waschschritt durchgeführt.

Neben der Bestimmung von IgE und de novo exprimiertem Oberflächenmolekül kann das erfindungsgemäße Verfahren auch die Bestimmung eines oder mehrerer zusätzlicher Parameter umfassen. So kann man die Probe zusätzlich mit einem dritten Bindemolekül inkubieren, das mit dem Oberflächenmolekül CD45 bindefähig ist, wobei das dritte Bindemolekül eine Markierungsgruppe trägt oder mit einer Markierungsgruppe bindefähig ist, die separat neben den Markierungsgruppen des ersten und zweiten Bindemoleküls nachweisbar ist. Das dritte Bindemolekül ist s vorzugsweise ein anti-CD45-Antikörper oder ein Fragment eines derartigen Antikörpers.

Durch zusätzliche Bestimmung von CD45, das nach Aktivierung der basophilen Granulozyten verstärkt exprimiert wird, kann die Genauigkeit der Bestimmung weiter erhöht werden.

Ebenfalls ein weiterer Parameter, der im erfindungsgemäßen Verfahren bestimmt werden kann, ist das Seitwärtsstreulicht, welches bei der Degranulation von Basophilen zunimmt.

Die im erfindungsgemäßen Verfahren verwendeten Bindemoleküle können eine Markierungsgruppe tragen oder mit einer Markierungsgruppe bindefähig sein. Wenn das Bindemolekül eine Markierungsgruppe trägt, d. h. direkt markiert ist, liegt es vorzugsweise als kovalentes Konjugat mit der Markierungsgruppe vor. Andererseits kann das Bindemolekül auch indirekt markiert sein, d. h. es trägt selbst direkt keine nachweisbare Markierungsgruppe sondern kann an eine weitere Substanz binden, die die Markierungsgruppe trägt. Beispiele für indirekt markierte Bindemoleküle sind Hapten-gekoppelte Antikörper, die mit einem eine Markierungsgruppe tragenden anti-Hapten-Antikörper bindefähig sind. Die Kopplung von Markierungsgruppen und Haptenen an biologische Bindemoleküle, insbesondere Antikörper oder Antikörperfragmente, ist dem Fachmann auf dem Gebiet immunologischer Testverfahren bekannt und braucht daher nicht näher erläutert zu werden. Für das erfindungsgemäße Verfahren sind direkt markierte Bindemoleküle bevorzugt.

Als Markierungsgruppen für die Bindemoleküle sind im wesentlichen alle auf dem Gebiet diagnostischer Nachweisverfahren bekannten Markierungen geeignet, wobei die einzige Voraussetzung darin besteht, daß die einzelnen verwendeten Markierungen nebeneinander bestimmbar sind. Bevorzugt sind Fluoreszenzmarkierungen mit jeweils einem unterschiedlichen spektralen Emissionsspektrum, z. B. Fluorescein, Phycoerythrin (PE) und Peridinium-Chlorophyll-A-Protein (Per CP) oder ein PE-Cyanin 5 Tandemkonjugat (z. B. Cychrome). Die Bestimmung wird bevorzugt in einem Durchflußzytometer ausgeführt.

Gemäß einer anderen bevorzugten Ausführungsform des vorliegenden Verfahrens wird als weiterer Parameter ein DNA-Farbstoff, der neben den Markierungen von anti-IgE-Antikörper und Rezeptor bestimmbar ist, zugegeben und bestimmt. Der DNA-Farbstoff dient zur Anfärbung der Zellkerne und somit dem Ausschluß von Thrombozyten und unlysierten Erythrozyten. Bevorzugt ist ein Fluoreszenzfarbstoff wie etwa 7-Aminoactinomycin D oder Propidiumjodid. Besonders bevorzugt ist 7-Aminoactinomycin D.

Die im erfindungsgemäßen Verfahren verwendete Probe ist eine Körperflüssigkeit, die Granulozyten enthält. Von besonderem Vorteil ist, daß das erfindungsgemäße Verfahren auf Vollblut angewendet werden kann, ohne daß eine Zellisolierung von basophilen Granulozyten erforderlich ist. Bevorzugt ist für die Probe heparinisiertes Vollblut und sie stammt im Allgemeinen von einem Humanspender, obwohl auch andere Proben von Säugern wie etwa Nagern eingesetzt werden können.

Das erfindungsgemäße Verfahren ist sehr einfach und schnell. Es kann an jedem handelsüblichen Durchflußcytometer durchgeführt werden, die in allen mittleren und größeren Krankenhäusern bzw. Labors vorhanden sind.

Die Verfahrensbedingungen können je nach verwendetem Testformat variiert werden. Als geeignete Bedingungen bei Verwendung von Fluoreszenzmarkierungen und einem durchflußzytometrischen Nachweis der Markierungen haben sich für Schritt (a), d. h. die Inkubation mit Testsubstanz, ein Zeitraum von 5 bis 60 Min. bevorzugt von 20 bis 40 Min. bei einer Temperatur von 25 bis 40°C, bevorzugt von 35 bis 39°C herausgestellt. Eine 30minütige Inkubation bei etwa 37°C ergibt gute Ergebnisse. Schritt (b) wird bevorzugt im Kalten durchgeführt, um eine unspezifische Degranulation zu vermeiden, wobei sich als geeignete Bedingungen eine Temperatur von 0 bis 10°C, bevorzugt eine Temperatur von 0 bis 5°C für eine Zeitdauer von 10 bis 40 Minuten, bevorzugt von 5 bis 30 Minuten herausgestellt haben.

Sofern nach Schritt (b) eine Fixierung durchgeführt wird, ist eine weitere Kühlung der Probe nicht essentiell, da durch die Fixierung eine weitere Degranulierung unterbunden wird. Schritt (c), d. h. die Bestimmung der Markierungen erfolgt im Anschluß an die Inkubationen sowie gegebenenfalls Lyse und nachfolgende Waschschritte. Dabei ist die Meßprobe für einen Zeitraum von mindestens einer Stunde, bevorzugt etwa zwei Stunden oder mehr, ausreichend stabil.

Wie für den Fachmann offensichtlich ist, werden bei einer Bestimmung nach dem erfindungsgemäßen Verfahren zweckmäßig geeignete Kontrollen mitgeführt, wobei als Negativ-Kontrolle ein Ansatz ohne Testsubstanz und als Positiv-Kontrolle eine die Granulozyten aktivierende Substanz wie beispielsweise fMLP geeignet ist. Weiterhin ist es günstig, eine Probe mitzuführen, deren Werte im wesentlichen im Normbereich liegen, wie etwa eine Körperflüssigkeit eines gesunden Normalprobanden.

Ein weiterer Gegenstand der vorliegenden Erfindung ist ein Reagenzienkit zur Bestimmung der Degranulationsaktivität von basophilen Granulozyten in einer Probe. Der Reagenzienkit enthält ein erstes Bindemolekül, das mit zellständigen IgE-Antikörpern oder deren hochaffinen Rezeptoren bindefähig ist und ein zweites Bindemolekül, das mit einem Oberflächenmolekül, welches bei Degranulation de novo an der Oberfläche von basophilen Granulozyten erscheint, bindefähig ist, wobei das erste und das zweite Bindemolekül unterschiedliche nebeneinander nachweisbare Markierungsgruppen tragen oder jeweils mit unterschiedlichen nebeneinander nachweisbaren Markierungsgruppen bindefähig sind. Gegebenenfalls kann der Reagenzienkit als weitere Nachweisreagenzien ein drittes Bindemolekül enthalten, das z. B. mit dem CD45 Oberflächenmolekül bindefähig ist und eine neben den Markierungen des ersten und zweiten Bindemoleküls nachweisbare Markierungsgruppe trägt bzw. mit einer solchen Markierungsgruppe bindefähig ist. Außerdem kann der Reagenzienkit auch einen DNA-Farbstoff enthalten. Weitere optionale Bestandteile des Reagenzienkits sind Testsubstanzen, d. h. verschiedene Allergene bzw. Positiv-Kontrollsubstanzen wie fMLP. Außerdem kann der Reagenzienkit auch noch Stimulantien wie Zytokine oder/und Reagenzien zur Lyse von Erythrozyten oder/und zur Fixierung von Leukozyten enthalten.

Weiterhin kann der Kit zusätzlich ein Reagenz zur Lyse von Erythrozyten oder/und zur Fixierung von Leukozyten sowie gegebenenfalls übliche Puffer, Hilfs- oder Zusatzstoffe enthalten.

Ein bevorzugter Reagenzienkit enthält beispielsweise mindestens eine Testsubstanz, einen anti-gp55-FITC-Antikörper, einen anti IgE-PE-Antikörper, eine FACS-Lysing Solution sowie gegebenenfalls 7-Aminoactinomycin D oder/und einen anti-CD45-PerCP-Antikörper und ein Stimulans wie etwa IL-3 oder GM-CSF.

Ein nochmals weiterer Gegenstand der Erfindung ist ein Verfahren zur Diagnose von allergischer Hypersensitivität eines Patienten bzw. eines Hyposensibilisierungserfolgs, welches dadurch gekennzeichnet ist, daß man die Degranulationsaktivität von basophilen Granulozyten in einer Probe aus einem Patienten durch das erfindungsgemäße Verfahren unter Verwendung von einem oder mehreren Allergenen und einer Positivkontrolle als Testsubstanzen bestimmt, in einem weiteren Ansatz die Degranulationsaktivität in einer negativen Kontrollprobe bestimmt und das Vorhandensein oder die Abwesenheit einer allergischen Hypersensitivität bzw. den Erfolg oder Mißerfolg einer Hyposensibilisierungstherapie durch Vergleich der erhaltenen Werte ermittelt.

Als negative Kontrollprobe dient zweckmäßig eine Probe aus dem selben Patienten, der keine Testsubstanz gemäß Schritt (a) des erfindungsgemäßen Verfahrens zugesetzt wurde, und bevorzugt wird als zweite negative Kontrollprobe eine stimulierte Probe eines gesunden Normalprobanden mitgeführt.

Ein weiterer Gegenstand dieser Erfindung ist die Verwendung von anti-gp55-Antikörpern in einem Verfahren zur Bestimmung von Degranulationsaktivität von basophilen Granulozyten sowie die Verwendung des erfindungsgemäßen Verfahrens bzw. Reagenzienkits bei der Diagnose von allergischer Hypersensitivität bzw. bei der Erfolgskontrolle einer Hyposensibilisierungstherapie.

Das erfindungsgemäße Verfahren wird weiter erläutert durch die nachfolgenden Beispiele in Verbindung mit den beigefügten Abbildungen 1-6. Es zeigen:
- Abb. 1:: die Auswertung eines erfindungsgemäßen Tests für eine Negativkontrolle,
- Abb. 2:: die Auswertung eines erfindungsgemäßen Tests für eine Positivkontolle,
- Abb. 3-5:: einen Vergleich erfindungsgemäßen Tests mit einem Histamin-Release-Test des Standes der Technik und
- Abb. 6:: die Auswertung eines erfindungsgemäßen Tests bei Vorstimulation mit IL-3.

### Beispiel 1

Peripheres Blut (heparinisiert) des Patienten wird mit unterschiedlichen Konzentrationen des suspekten Allergens bei 37°C inkubiert. Dadurch kommt es bei entsprechender Sensibilisierung zu einer Degranulation der basophilen Granulozyten und zu einer Aktivierung, die vor allem durch Expression des gp55-Antigens gekennzeichnet ist. Weiterhin sinkt die Expressionsdichte des IgE-Rezeptors und die Dichte von CD45 auf Basophilen steigt. Das Seitwärtsstreulicht steigt (Verlust der absorbierenden Granula) und die Anzahl der Basophilen im Streulichtgate für Lymphozyten verringert sich. Der Nachweis erfolgt durch eine Dreifarbimmunfluoreszenz und durchflußzytometrische Analyse. Als Positivkontrolle dient das chemotaktische Peptid fMLP, als Negativkontrolle Pufferlösung (PBS). Die Einnahme von Antihistaminika hemmt den Test.

### 1. Materialien

### 1.1 Reagenzien

Sheathflüssikgeit: FACS-Flow (Fa. Becton Dickinson, Best-Nr. 342003) oder eine andere Trägerflüssigkeit mit niedriger Eigenfluoreszenz.

FACS-Lysing Solution, 10x-Konzentrat (Fa. Becton Dickinson, Best-Nr. 349202),

Waschlösung: PBS (Phosphate buffered saline ohne Calcium und Magnesium) als fertige Lösung oder Salze (z.B. Biochrom, Berlin), oder PHAGO-BURST Waschlösung (Fa. ORPEGEN Pharma, Heidelberg).

Chemotaktisches Peptid
fMLP (2000x) (Fa. ORPEGEN), in PBS verdünnen.

Zur Kalibration bei quantitativen Messungen:
Calibrite Eichpartikel, Becton Dickinson, Best-Nr.: 349502

### 1.2 Monoklonale Antikörper

a) anti gp55-FITC, (ORPEGEN Pharma), Bestell-Nr. 1464 (in der fertigen Kombination 1:4 vorverdünnt)
b) anti IgE-PE (ORPEGEN Pharma); Bestell-Nr. 1465 (in der fertigen Kombination 1:40 vorverdünnt)
c) anti CD45 PerCP (Becton-Dickinson) oder CD45-Cychrome (Pharmingen), Bestell-Nr.

### 1.3 Allergene

ALK-Depot SQ (Scherax) 6-Gräsermischung + Roggen 100.000 U/ml, Bestell-Nr. 145a/90.90c
Latex Allergen (ALYOSTAL ST-IR) Bestell-Nr. 0903
ALK-prick SQ (Scherax) Bienengift, Zul.-Nr. 222a/85a, 300µg/ml
ALK-prick SQ (Scherax) Wespengift, Zul.-Nr. 223a/85a, 300µg/ml Allergopen (Penicillin G, PPL und Derviat MDM), Zul.-Nr. 162a/81,
Penicillin "Grünenthal" 1 Mega, PZN-7803133
Milbenallergen der Firma Geer-Labs u.a.m.

### 2. Durchführung

### 2.1 Probenvorbereitung

Die zu untersuchende Probe wird in Aliquots von jeweils 100 µl aufgeteilt. Bei höheren Leukozytenzahlen (WBC von 10-20000 Z/µl) kann ein geringeres Probenvolumen z. B. 50 µl verwendet werden. Mehrfachansätze sind nicht erforderlich.

### 2.2 Vorbereitung der Allergenverdünnungen

ALK-Depot SQ (Scherax) 6-Gräsermischung + Roggen wird in Konzentrationen von 10.000, 1.000, 100 und 10 U/ml eingesetzt. ALK-prick SC (Scherax) Bienengift und ALK-prick SQ (Scherax) Wespengift werden in Konzentrationenen von 10, 1, 0,1 und 0,01 µg/ml eingesetzt. Latexallergen, PPL (Allergopen) und MDM (Allergopen) werden in Verdünnungen von 1:10, 1:100 und 1:1.000 eingesetzt. Penicillin Grünenthal wird in Konzentrationen von 100, 10, 1 und 0,1 µg/ml eingesetzt. Als Negativ-Kontrolle dient PBS und als Positivkontrolle wird fMLP in einer Konzentration von 10⁻⁷ M (d.h. 1:2.000 PBS) verwendet. Als weitere Kontrolle wird eine Probe aus einem gesunden Normalprobanden mitgeführt.

### 2.3 Inkubation mit Allergen

Je 100 µl der Allergenverdünnung werden zu einem Aliquot der Probe gegeben, gemischt und 30 Minuten bei 37°C im Wasserbad inkubiert. Dann werden die Ansätze auf Eis gestellt.

### 2.4 Zugabe der Antikörper

Dann werden folgende Antikörperlösungen zugegeben.
a) 20 µl anti gp55-FITC (vorverdünnt)
b) 20 µl anti IgE-PE (vorverdünnt)
c) 10 µl anti CD45-Cychrome bzw. 20 µl CD45-PerCP

Nach der Antikörperzugabe wird gut gemischt (Vortex) und für 20 Minuten lichtgeschützt auf Eis inkubiert.

### 2.5 Lyse und Fixierung

Dann erfolgt eine Zugabe von 2 ml FACS-Lysing-Solution (Erythrozytenlyse und Leukozytenfixation), Mischen (VORTEX) und Inkubation 10 Minuten lichtgeschützt bei Raumtemperatur. Anschließend wird 5 min bei 1300 U/min bzw. 300 g abzentrifugiert und dekantiert.

### 2.6 Waschen der Zellen

Das Waschen der Zellen erfolgt durch Zugabe von 3 ml Waschlösung (PBS), Abzentrifugieren (5 min 1300 U/min bzw. 300 g) und Dekantieren.

### 2.7 Messung

Die Messung der Probe erfolgt durchflußzytometrisch, z. B. an einem FACScan Gerät. Die Stabilität der Meßprobe berägt vermutlich ≥ 2 Stunden auf Flockeneis im Dunkeln.

Die Geräteeinstellung ("BASO-SET" im Programm CellQuest®) kann wie folgt gewählt werden.

| Parameter | Verstärker | Erwartungswert | Auflösung |
|---|---|---|---|
| FSC | lin | E00 (2,0) | 256 Kanäle |
| SSC | lin | 360 | 256 Kanäle |
| FL1 | log, 4 Dekaden | 620 | 256 Kanäle |
| FL2 | log, 4 Dekaden | 500 | 256 Kanäle |
| FL3 | log, 4 Dekaden | 620 | 256 Kanäle |
| Trigger auf FSC | | 100 | |
| Kompensation | FL1 - %FL2 | 0,9 | |
| | FL2-%FL1 | 21 | |
| | FL2 - %FL3 | 0,2 | |
| | FL3 - %FL2 | 29 | |

| - Filterkombinationen (im FACScan nicht variabel): | | | | |
|---|---|---|---|---|
| Parameter: | | Spektrum | Fluorochrom | Filter |
| FL1 | Fluoreszenz 1 | Grünfluoreszenz | FITC, TO | BP 530 +/- 15 nm |
| FL2 | Fluoreszenz 2 | Orangefluoreszenz | PE, PI | BP 585 +/- 22 nm |
| FL3 | Fluoreszenz 3 | Rotfluoreszenz | PerCP, Red613, Cy5, PI | LP > 650 nm |

Es wird im Dotplot SSC/IgE eine Region um die Zellen mit geringem und hohem Seitwärtsstreulicht und starker IgE-Expression gesetzt. Diese Zellen werden optional im Rahmen der Validierung im Dotplot FL3 (CD45) vs FL2 (IgE) dargestellt und die x- bzw. y-Meanwerte dieser Population ausgewertet, weiterhin das FL1-Histogramm (gp55). Die Marker werden so gesetzt, daß die Auswerteregion den Bereich der gp55 positiven Basophilen erfasst (als Negativkontrolle dient der PBS Ansatz). Der Meanwert FL1 wird von allen (auch gp55 negativen) Zellpopulationen betrachtet. Es werden mög-lichst mindestens 1.000 Zellen von der interessierenden Subklasse (hier: Basophile) aufgenommen. Im positiven Ansatz entsteht eine bimodale Verteilung, in der die aktivierten basophilen Granulozyten eine eigene Population darstellen. Es kann daher der prozentuale Anteil betrachtet werden, was für die Bewertung des Testergenisses ausreichend ist.

### 3. Auswertung

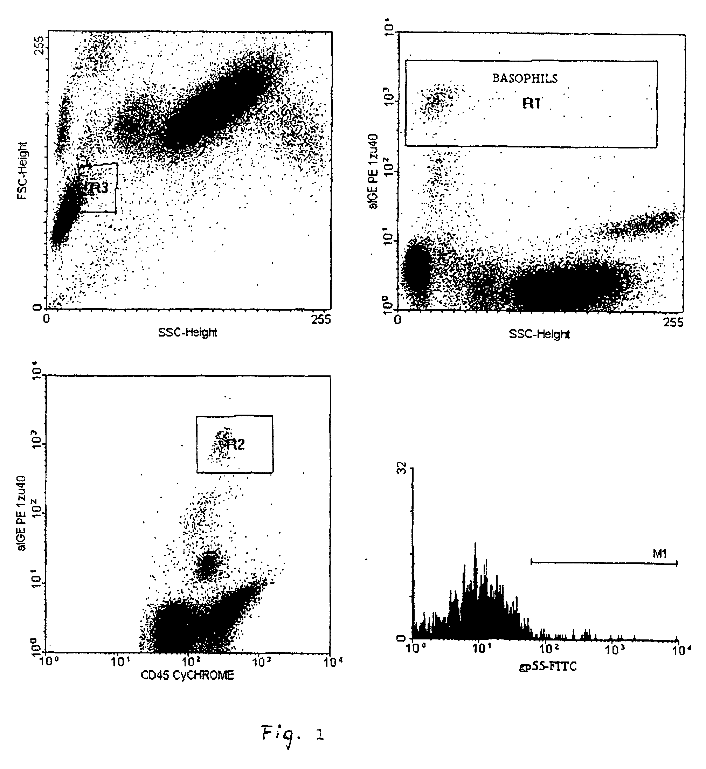

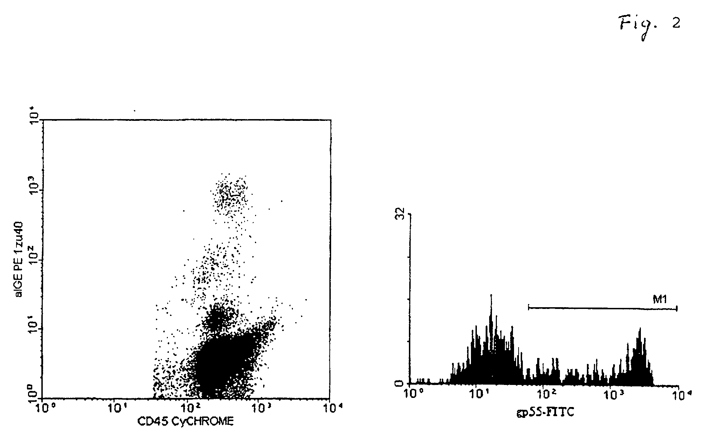

Die Auswertung für eine Negativkontrolle mit PBS ist in Abb. 1 dargestellt. Die Auswertung für eine Positivkontrolle mit fMLP ist in Abb. 2 dargestellt.

Die nachstehende Tabelle 1 zeigt typische Werte, wobei aus Plausibilitätsgründen alle Parameter zu berücksichtigen sind. Ein Vergleich zwischen Negativkontrolle (PBS) und Positivkontrolle (fMLP) zeigt, daß der Prozentsatz der Basophilen im wesentlichen stabil bleibt, die CD45 Expressionsdichte nach Aktivierung steigt und die IgE-Dichte leicht sinkt. Der Prozentsatz der gp55 positiven basophilen Granulozyen sowie der Mean gp55 Wert nimmt nach Aktivierung stark zu.

| Ansatz | %Basos | Mean CD45 | Mean IgE | %gp55 | Mean gp55 |
|---|---|---|---|---|---|
| PBS | 2,50 | 134 | 1032 | 3,3 | 33 |
| fMLP | 2,35 | 349 | 981 | 30,6 | 369 |

Ein Vergleich der Diagramme für CD 45 und gp55 bei der Positiv- und Negativkontrolle zeigt, daß für gp55 eine deutlich höhere allergenspezifisch induzierte Erhöhung der Expression stattfindet.

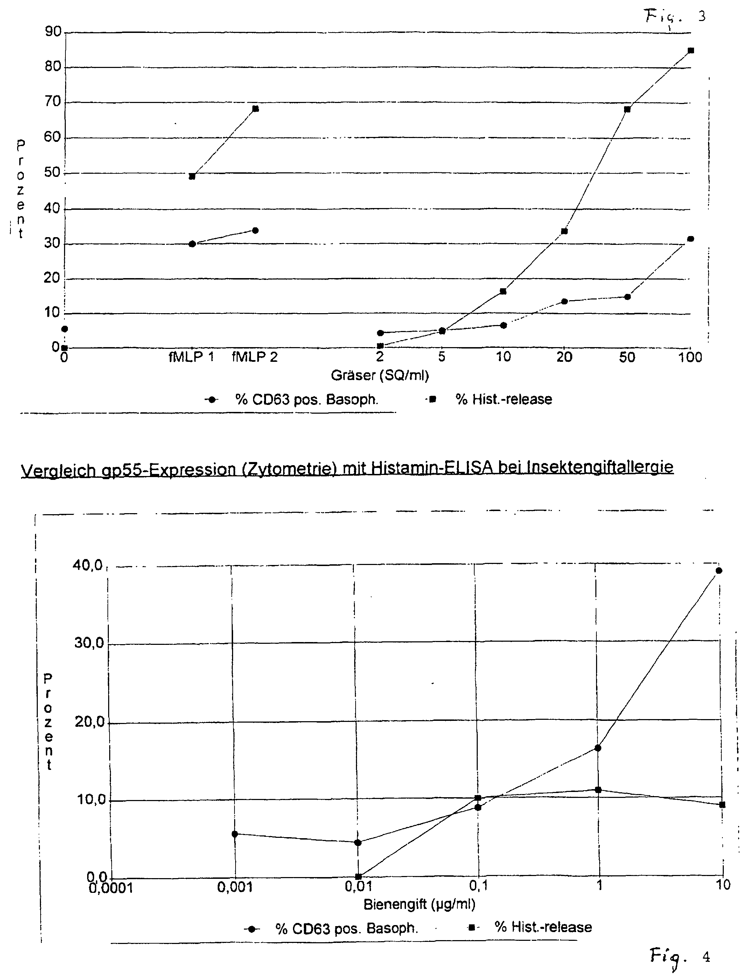

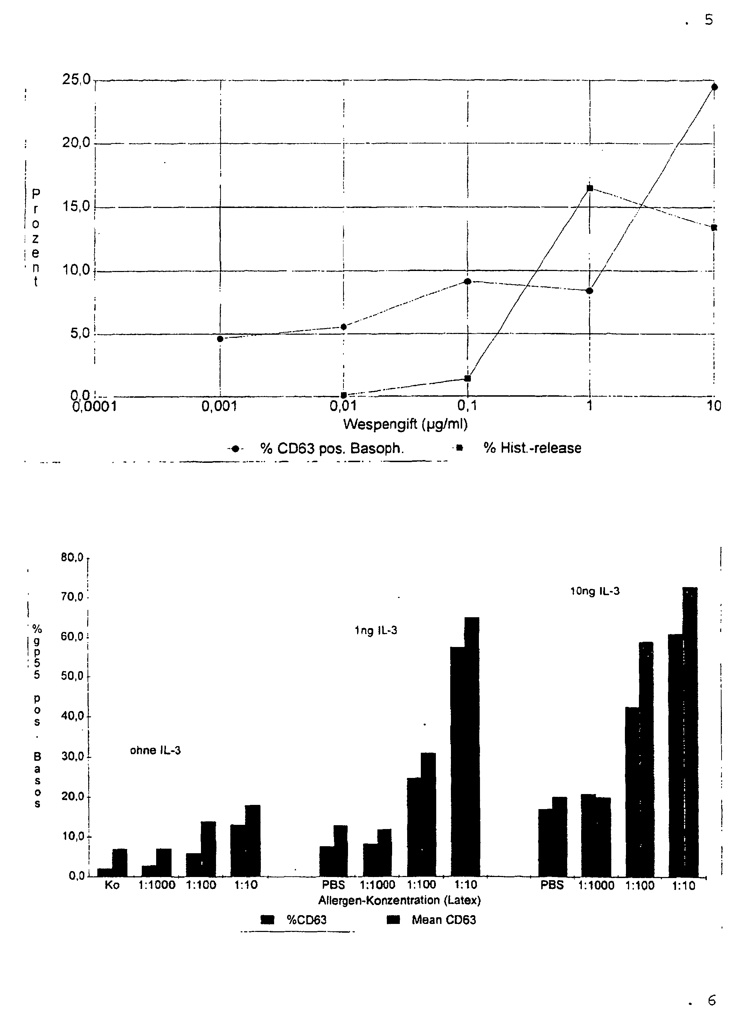

In den Abb. 3-6 sind Resultate des erfindungsgemäßen Verfahrens dargestellt. Abb. 3 zeigt einen Verlgeich des erfindungsgemäßen Verfahrens (gp55 bzw. CD63-Expression) mit dem herkömmlichen Histamin-Release-Test bei der Bestimmung einer Gräserpollenallergie. Entsprechende Daten sind in Abb. 4 für die Bestimmung einer Bienengiftallergie bzw. in Abb. 5 für die Bestimmung einer Wespengiftallergie gezeigt. Aus Abb. 6 ist am Beispiel einer Latexallergenbestimmung ersichtlich, daß durch Vorstimulation mit einem Zytokin wie IL-3 die Degranulationsaktivität der Basophilen deutlich verstärkt werden kann.

### Legende:

FSC = forward light scatter = narrow angle forward light scatter = Zellgröße
SSC = side scatter = orthogonal light scatter = Zellgranularität
algE-PE = IgE-PE = monoklonaler Antikörper gegen das Immunglobulin E, direkt konjugiert mit dem Fluoreszenzfarbstoff Phycoerythrin.
gp55-FITC = monoklonaler Antikörper gegen das lysosomale Glykoprotein mit dem Molekulargewicht 55 kD, direkt konjugiert mit dem Fluoreszenzfarbstoff Fluorescein.
CD45 CyChrome = monoklonaler Antikörper gegen das gemeinsame Zelloberflächen-Glykoprotein von Leukozyten, direkt konjugiert mit dem Tandemfarbstoff PE-Cyanin 5
R1, R2, R3 = Regionen in denen bestimmte Zellen liegen und die als logisches Gate funktionieren. In diesem Falle die typischen Regionen für basophile Granulozyten
M1 = Markerregion in der die für das Antigen gp55 positiv reagierenden Zellen liegen (degranulierte Basophile)

## Patentansprüche

1. Verfahren zur Bestimmung der durch eine Testsubstanz ausgelöste Degranulationsaktivität von basophilen Granulozyten in einer Probe,
**dadurch gekennzeichnet,**
**daß** man
(a) die Probe mit einer Testsubstanz vermischt und inkubiert,
(b) die Probe mit einem ersten Bindemolekül, das mit zellständigen IgE Antikörpern oder deren hochaffinen Rezeptoren bindefähig ist, und einem zweiten Bindemolekül, das mit einem Oberflächenmolekül, welches bei Degranulation de novo an der Oberfläche von basophilen Granulozyten erscheint, bindefähig ist, vermischt und inkubiert, wobei das erste Bindemolekül und das zweite Bindemolekül unterschiedliche nebeneinander nachweisbare Markierungsgruppen tragen oder jeweils mit unterschiedlichen nebeneinander nachweisbaren Markierungsgruppen bindefähig sind, und
(c) die Markierungen der Bindemoleküle separat bestimmt und auf Basis dieser Bestimmung die Degranulationsaktivität in der Probe qualitativ oder/und quantitativ ermittelt.

2. Verfahren nach Anspruch 1,
**dadurch gekennzeichnet,**
**daß** man die Probe mit einem die Degranulationsaktivität verstärkenden Stimulans vorinkubiert.

3. Verfahren nach Anspruch 2,
**dadurch gekennzeichnet,**
**daß** man als Stimulans ein Zytokin, insbesondere IL-3 oder GM-CSF nimmt.

4. Verfahren nach den Ansprüchen 1 bis 3,
**dadurch gekennzeichnet,**
**daß** man als erstes Bindemolekül oder/und als zweites Bindemolekül einen Antikörper oder ein Antikörperfragment verwendet.

5. Verfahren nach einem der vorhergehenden Ansprüche,
**dadurch gekennzeichnet,**
**daß** man als erstes Bindemolekül einen anti-IgE-Antikörper oder ein Fragment davon oder einen anti-Fc_{∈} R1-Antikörper oder ein Fragment davon verwendet.

6. Verfahren nach einem der vorhergehenden Ansprüche,
**dadurch gekennzeichnet,**
**daß** man als zweites Bindemolekül einen anti-gp55-Antikörper oder ein Fragment davon verwendet.

7. Verfahren nach einem der vorhergehenden Ansprüche,
**dadurch gekennzeichnet,**
**daß** man vor Schritt (c) Erythrozyten lysiert oder/und Leukozyten fixiert.

8. Verfahren nach einem der vorhergehenden Ansprüche,
**dadurch gekennzeichnet,**
**daß** man direkt markierte Bindemoleküle verwendet.

9. Verfahren nach einem der vorhergehenden Ansprüche,
**dadurch gekennzeichnet,**
**daß** die Bindemoleküle nebeneinander nachweisbare Fluoreszenzmarkierungen tragen.

10. Verfahren nach Anspruch 9,
**dadurch gekennzeichnet,**
**daß** man die Bestimmung in einem Durchflußzytometer durchführt.

11. Verfahren nach einem der vorhergehenden Ansprüche,
**dadurch gekennzeichnet,**
**daß** man die Probe zusätzlich mit einem DNA-Farbstoff, der ein Fluoreszenzfarbstoff ist und der neben den Markierungen der Bindemoleküle nachweisbar ist, inkubiert.

12. Verfahren nach Anspruch 11,
**dadurch gekennzeichnet,**
**daß** der fluoreszierende DNA-Farbstoff 7-Aminoactinomycin D ist.

13. Verfahren nach einem der vorhergehenden Ansprüche,
**dadurch gekennzeichnet,**
**daß** die Probe Vollblut ist.

14. Verfahren nach einem der vorhergehenden Ansprüche,
**dadurch gekennzeichnet,**
**daß** man die Inkubation von Schritt (a) während 5-60 min bei einer Temperatur von 25-40°C durchführt, insbesondere während 20-40 min bei 35-39°C durchführt.

15. Verfahren nach einem der vorhergehenden Ansprüche,
**dadurch gekennzeichnet,**
**daß** man die Inkubation von Schritt (b) während 10-40 min bei einer Temperatur von 0-10°C durchführt.

16. Reagenzienkit zur Bestimmung der Degranulationsaktivität von basophilen Granulozyten,
**dadurch gekennzeichnet,**
**daß** er mindestens ein erstes Bindemolekül, das mit zellständigen IgE-Antikörpern bindefähig ist, und ein zweites Bindemolekül, das mit einem Oberflächenmolekül, welches bei Degranulation de novo an der Oberfläche von basophilen Granulozyten erscheint, bindefähig ist, enthält, wobei das erste und das zweite Bindemolekül unterschiedliche nebeneinander nachweisbare Markierungsgruppen tragen oder jeweils mit unterschiedlichen nebeneinander nachweisbaren Markierungsgruppen bindefähig sind.

17. Reagenzienkit nach Anspruch 16,
**dadurch gekennzeichnet,**
**daß** er zusätzlich enthält: (a) ein drittes Bindemolekül, das mit dem CD45-Oberflächenmolekül bindefähig ist, wobei das dritte Bindemolekül eine mit den Markierungsgruppen des ersten und zweiten Bindemoleküls nachweisbare Markierungsgruppe trägt oder damit bindefähig ist, (b) einen DNA-Farbstoff, (c) ein Reagenz zur Lyse von Erythrozyten oder/und zur Fixierung von Leukozyten, (d) mindestens eine Testsubstanz, einen anti-gp55-FITC Antikörper, anti-IgE-PE Antikörper, FACS-Lysing Solution sowie gegebenenfalls 7-Aminoactinomycin D oder/und einen anti-CD45-PerCP Antikörper oder/und ein Stimulans, oder/und (e) übliche Puffer, Hilfs- oder/und Zusatzstoffe.

18. Verfahren zur Diagnose von allergischer Hypersensitivität eines Patienten, bzw. eines Hyposensibilisierungserfolgs,
**dadurch gekennzeichnet,**
**daß** man die Degranulationsaktivität von basophilen Granulozyten in einer Probe aus dem Patienten durch ein Verfahren nach einem der Ansprüche 1 bis 15 unter Verwendung von einem oder mehreren Allergenen und einer Positivkontrolle als Testsubstanzen bestimmt, in einem weiteren Ansatz die Degranulationsaktivität in einer negativen Kontrollprobe bestimmt und das Vorhandensein oder die Abwesenheit einer Hypersensitivität bzw. den Erfolg oder den Mißerfolg einer Hyposensibilisierungstherapie durch Vergleich der erhaltenen Werte ermittelt.

19. Verwendung von anti-gp55 Antikörpern in einem Verfahren zur Bestimmung der Degranulationsaktivität von basophilen Granulozyten.

20. Verwendung eines Verfahrens nach einem der Ansprüche 1 bis 15 bei der Diagnose von allergischer Hypersensitivität.

21. Verwendung eines Reagenzienkits nach einem der Ansprüche 16 oder 17 bei der Diagnose von allergischer Hypersensitivität.

22. Verwendung eines Verfahrens nach einem der Ansprüche 1 bis 15 bei der Erfolgskontrolle einer Hyposensibilisierungstherapie.

23. Verwendung eines Reagenzienkits nach einem der Ansprüche 1 bis 17 bei der Erfolgskontrolle einer Hyposensibilisierungstherapie.

## Claims

1. Method for the determination of the degranulation activity triggered by a test substance of basophilic granulocytes in a sample, **wherein**
a) the sample is mixed and incubated with a test substance,
b) the sample is mixed and incubated with a first binding molecule which can bind to cell-bound IgE antibodies or high affinity receptors thereof, and with a second binding molecule which can bind to a surface molecule which appears de novo on the surface of basophilic granulocytes during degranulation, where the first binding molecule and the second binding molecule carry different marker groups that can be detected concurrently or can each bind to different marker groups that can be detected concurrently, and
c) the labels of the binding molecules are determined separately and the degranulation activity in the sample is determined qualitatively or/and quantitatively on the basis of this determination.

2. Method as claimed in claim 1,
**wherein**
the sample is preincubated with a stimulant that amplifies the degranulation activity.

3. Method as claimed in claim 2,
**wherein**
a cytokine, in particular IL-3 or GM-CSF is used as a stimulant.

4. Method as claimed in claims 1 to 3,
**wherein**
an antibody or an antibody fragment is used as the first binding molecule or/and as the second binding molecule.

5. Method as claimed in one of the previous claims,
**wherein**
an anti-IgE antibody or a fragment thereof or an anti-Fc_{∈} R1 antibody or a fragment thereof is used as the first binding molecule.

6. Method as claimed in one of the previous claims,
wherein
an anti-gp55 antibody or a fragment thereof is used as the second binding molecule.

7. Method as claimed in one of the previous claims,
wherein
erythrocytes are lysed or/and leukocytes are fixed before step (c).

8. Method as claimed in one of the previous claims,
**wherein**
directly labelled binding molecules are used.

9. Method as claimed in one of the previous claims,
**wherein**
the binding molecules carry fluorescent markers that can be detected concurrently.

10. Method as claimed in claim 9,
**wherein**
the determination is carried out in a flow cytometer.

11. Method as claimed in one of the previous claims,
**wherein**
the sample is additionally incubated with a DNA dye which is a fluorescent dye and can be detected in addition to the binding molecule labels.

12. Method as claimed in claim 11,
wherein
the fluorescent DNA dye is 7-aminoactinomycin D.

13. Method as claimed in one of the previous claims,
wherein
the sample is whole blood.

14. Method as claimed in one of the previous claims,
wherein
the incubation of step (a) is carried out for 5 - 60 min at a temperature of 25-40°C, in particular for 20 -40 min at 35-39°C.

15. Method as claimed in one of the previous claims,
wherein
the incubation of step (b) is carried out for 10-40 min at a temperature of 0-10°C.

16. Reagent kit for the determination of the degranulation activity of basophilic granulocytes,
wherein
it contains at least one first binding molecule which can bind to cell-bound IgE antibodies and a second binding molecule which can bind to a surface molecule which appears de novo on the surface of basophilic granulocytes during degranulation where the first and the second binding molecule carry different marker groups that can be detected concurrently or each can bind to different marker groups that can be detected concurrently.

17. Reagent kit as claimed in claim 16,
wherein
it additionally contains a third binding molecule which can for example bind to the CD45 surface molecule and carries a marker group which can be detected in addition to the markers of the first and second binding molecules or can bind to such a marker group, (b) a DNA dye, (c) a reagent for lysing erythrocytes or/and for fixing leukocytes, (d) at least one test substance, an anti-gp55 FITC antibody, anti-IgE-PE antibody, FACS lysing solution as well as optionally 7-aminoactinomycin D or/and an anti-CD45-PerCP antibody or/and a stimulant, or/and (e) conventional buffers, auxiliary substances or/and additives.

18. Method for diagnosing allergic hypersensitivity of a patient or the response to a hyposensitization,
wherein
the degranulation activity of basophilic granulocytes is determined in a sample from the patient by means of a method according to the invention as claimed in one of the claims 1 to 15 using one or several allergens and a positive control as test substances, the degranulation activity in a negative control sample is determined in an additional test mixture and the presence or the absence of an allergic hypersensitivity or the success or failure of a hyposensitization therapy is determined by comparing the values that are obtained.

19. Use of anti-gp55 antibodies in a method for determining the degranulation activity of basophilic granulocytes.

20. Use of a method as claimed in one of the claims 1 to 15 for diagnosing allergic hypersensitivity.

21. Use of a reagent kit as claimed in one of the claims 16 to 17 for diagnosing allergic hypersensitivity.

22. Use of a method as claimed in one of the claims 1 to 15 for monitoring the response to a hyposensitization therapy.

23. Use of a reagent kit as claimed in one of the claims 1 to 17 for monitoring the response to a hyposensitization therapy.

## Revendications

1. Procédé pour la détermination de l'activité de dégranulation déclenchée par une substance test de granulocytes basophiles dans un échantillon, **caractérisé en ce que** :
(a) on mélange et incube l'échantillon avec une substance test,
(b) l'échantillon peut être relié avec une première molécule de liaison, qui peut être reliée avec des anticorps/IgE stables au niveau de la cellule ou leurs récepteurs très affines, et une deuxième molécule de liaison, qui peut être reliée, mélangée et incubée avec une molécule de surface, qui apparaît en cas de dégranulation de novo à la surface de granulocytes basophiles, la première molécule de liaison et la deuxième molécule de liaison portant des groupes de marquage différents et décelables de façon juxtaposée ou pouvant être reliées respectivement avec des groupes de marquage différents et décelables de façon juxtaposée, et
c) les marquages des molécules de liaison sont déterminés séparément et l'activité de dégranulation dans l'échantillon est déterminée au plan qualitatif et/ou quantitatif sur la base de cette détermination.

2. Procédé selon la revendication 1, **caractérisé en ce qu'**on fait préincuber l'échantillon avec un stimulant qui renforce l'activité de dégranulation.

3. Procédé selon la revendication 2, **caractérisé en ce qu'**on prend comme stimulant un cytokine, en particulier IL-3 ou GM-CSF.

4. Procédé selon les revendications 1 à 3, **caractérisé en ce qu'**on utilise comme première molécule de liaison ou/et comme molécule liaison un anticorps ou un fragment d'anticorps.

5. Procédé selon l'une quelconque des revendications précédentes, **caractérisé en ce qu'**on utilise comme première molécule de liaison un anticorps anti-IgE ou un fragment de celui-ci ou un anticorps R1 anti-FCE ou un fragment de celui-ci.

6. Procédé selon l'une quelconque des revendications précédentes, **caractérisé en ce qu'**on utilise comme deuxième molécule de liaison un anticorps anti-gp55 ou un fragment de celui-ci.

7. Procédé selon l'une quelconque des revendications précédentes, **caractérisé en ce qu'**on lyse des érythrocytes ou/et fixe des leucocytes avant l'étape (c).

8. Procédé selon l'une quelconque des revendications précédentes, **caractérisé en ce qu'**on utilise des molécules de liaison marquées directement.

9. Procédé selon l'une quelconque des revendications précédentes, **caractérisé en ce que** les molécules de liaison portent des repères de fluorescence décelables de façon juxtaposée.

10. Procédé selon la revendication 9, **caractérisé en ce qu'**on effectue la détermination dans un cytomètre d'écoulement.

11. Procédé selon l'une quelconque des revendications précédentes, **caractérisé en ce qu'**on incube l'échantillon en supplément avec un colorant pour l'ADN, qui est un colorant de fluorescence et peut être décelé parallèlement aux repères des molécules de liaison.

12. Procédé selon la revendication 11, **caractérisé en ce que** le colorant pour l'ADN fluorescent est de la 7-aminoactinomycine D.

13. Procédé selon l'une quelconque des revendications précédentes, **caractérisé en ce que** l'échantillon est du sang pur.

14. Procédé selon l'une quelconque des revendications précédentes, **caractérisé en ce qu'**on effectue l'incubation de l'étape (a) pendant 5 à 60 minutes à une température de 25 à 40°C, en particulier pendant 20 à 40 minutes à une température de 35 à 39 °C.

15. Procédé selon l'une quelconque des revendications précédentes, **caractérisé en ce qu'**on effectue l'incubation de l'étape (b) pendant 10 à 40 minutes à une température de 0 à 10°C.

16. Kit de réactifs pour déterminer l'activité de dégranulation de granulocytes basophiles, **caractérisé en ce qu'**il contient au moins une première molécule de liaison, qu'il peut être liée avec des anticorps/IgE stables au niveau de la cellule, et une deuxième molécule de liaison, qui peut être liée avec une molécule de surface, laquelle apparaît en cas de dégranulation de novo à la surface de granulocytes basophiles, les première et seconde molécules de liaison portant des groupes de marquage différents et décelables de façon juxtaposée ou peuvent être liés respectivement avec des groupes de marquage différents et décelables de façon juxtaposée.

17. Kit de réactifs selon la revendication 16, **caractérisé en ce qu'**il contient en supplément : (a) une troisième molécule de liaison, qui peut être liée avec la molécule de surface de CD45, la troisième molécule de liaison portant un groupe de marquage décelable avec les groupes de marquage des première et seconde molécules de liaison ou pouvant être liée avec ce groupe (b) un colorant pour l'ADN, (c) à réactif pour la lyse d'érythrocytes ou/et pour la fixation de leucocytes, (d) au moins une substance test, un anticorps anti-gp55-FITC, des anticorps anti-Ige-PE, une FACS-Lysing Solution et éventuellement de la 7-aminoactinomycne D ou/et un anticorps anti-ICD45-PerCP ou/et un stimulant ou/et (e) des produits tampons, auxiliaires ou/et supplémentaires classiques.

18. Procédé pour le diagnostic de l'hypersensibilité allergique d'un patient ou d'un résultat d'hyposensibilisation, **caractérisé en ce qu'**on détermine l'activité de dégranulation de granulocytes basophiles dans un échantillon provenant du patient par un Procédé défini selon l'une quelconque des revendications 1 à 15 en utilisant un ou plusieurs allergènes et un contrôle positif comme substances tests, on détermine dans une autre évaluation l'activité de dégranulation dans un échantillon de contrôle négatif et on détermine la présence ou l'absence d'une hypersensibilité respectivement la réussite ou l'échec d'une thérapie d'hyposensibilisation par la comparaison des résultats obtenus.

19. Utilisation d'anticorps anti-gp55 dans un Procédé pour déterminer l'activité de dégranulation de granulocytes basophiles.

20. Utilisation d'un Procédé selon l'une quelconque des revendications 1 à 15 pour le diagnostic d'une hypersensibilité allergique.

21. Utilisation d'un kit de réactifs selon l'une quelconque des revendications 16 ou 17 pour le diagnostic d'une hypersensibilité allergique.

22. Utilisation d'un Procédé selon l'une quelconque des revendications 1 à 15 pour le contrôle du résultat d'une thérapie d'hyposensibilisation.

23. Utilisation d'un kit de réactifs selon l'une quelconque des revendications 1 à 17 pour le contrôle du résultat d'une thérapie d'hyposensibilisation.
